# EUROPEAN PATENT SPECIFICATION

(11) **EP 3 389 497 B1**
(45) Date of publication and mention of the grant of the patent: **09.12.2020**
(21) Application number: 16810402.4
(22) Date of filing: 16.12.2016
(51) Int. Cl.: A61B 6/03, A61B 6/00, G06K 9/62, G06T 7/00, G06T 7/40, G06K 9/00

(54) **METHOD AND DEVICE FOR A MEDICAL IMAGE ANALYSIS**
VERFAHREN ZUR OPTIMIERUNG EINES VERFAHRENS ZUR VERARBEITUNG MEDIZINISCHER BILDER
PROCÉDÉ D'OPTIMISATION D'UN PROCÉDÉ DE TRAITEMENT D'IMAGE MÉDICALE

(30) Priority: 17.12.2015 EP 15200869
(43) Date of publication of application: 24.10.2018
(73) Proprietor: Koninklijke Philips N.V., 5656 AG Eindhoven (NL)
(72) Inventor: CARMI, Raz, 5656 AE Eindhoven (NL)
(74) Representative: Versteeg, Dennis John
(86) International application number: PCT/EP2016/081331
(87) International publication number: WO 2017/103037

(56) References cited:
- WO-A1-2006/090321
- US-A1- 2015 297 166
- US-B1- 6 574 304

## Description

The present invention relates to a method and a device for a medical image analysis. The invention further relates to a respective computer program element and a respective computer readable medium.

### BACKGROUND OF THE INVENTION

The field of 'Radiomics' usually refers to the extraction and analysis of large amounts of information from medical images using advanced quantitative feature analysis, for example in the context of comprehensive quantification of tumor phenotypes. The image feature space, corresponding to the relevant selected regions (such as a segmented tumor), is constructed using automatically extracted data-characterization algorithms.

A central hypothesis in Radiomics, that has been recently validated in clinical studies, is that the underlying biological characteristics of aggressive tumors could translate into macroscopic heterogeneous tumor metabolism and anatomy. Some studies reveal that a prognostic Radiomic signature, related to tumor tissue heterogeneity and other imaging phenotypes, is correlated with tumor stage, metabolism, hypoxia, angiogenesis and the underlying gene and/or protein expression profiles. These results suggest that Radiomics, with its analyzed imaging features, identifies a general prognostic phenotype existing in multiple cancer types and may thus show clinical significance across different diseases.

One key goal of imaging is 'personalized medicine', where treatment is increasingly tailored on the basis of specific characteristics of the patient. Much of the discussion of personalized medicine has focused on molecular characterization using genomic and proteomic technologies. However, as tumors are spatially and temporally heterogeneous, these techniques are limited. They require biopsies or invasive surgeries to extract and analyze what are generally small portions of tumor tissue, which do not allow for a complete characterization of the tumor.

Medical imaging modalities such as Computed Tomography, Magnetic Resonance Imaging and Positron Emission Tomography have great potential to guide therapy because it can provide a more comprehensive view of the entire tumor and it can be used on an ongoing basis to monitor the development and progression of the disease or its response to therapy. Further, imaging is noninvasive and is already often repeated during treatment in routine practice. Radiomics can have a large clinical impact providing a method that can quantify and monitor phenotypic changes during treatment and reveal important prognostic information about disease risk in practical workflow and at low cost.

It is known to apply many Radiomics features to the selected image volume region. At a second step, an algorithm is applied to find a smaller set of meaningful or significant features. Then, a machine learning approach is sometimes used to obtain classification results with respect to known clinical conditions. Common image features are usually related to mathematical classes such as pixel value statistics, histogram analysis, texture analysis, gray-level co-occurrence matrix approach (GLCM), wavelet analysis, fractal analysis, and various types of shape and fine-structure analysis.

In order that medical images, such as Computed Tomography data, will be able to provide high quality Radiomics analysis, it is crucial to achieve high spatial resolution, low image noise, and high low-contrast detectability for discriminating between different soft tissue types. This very often contradicts common clinical Computed Tomography protocols in which reducing radiation dose is a highly important factor.

For example abdomen Computed Tomography standard scan protocols are designed for dose saving. The final images usually have a resolution of 8-10 line-pairs per centimeter (lp/cm) and image slice width of 1.5 - 3.0 mm. The x-ray tube voltage is usually 100-120 kVp, which is not always optimal for the highest low-contrast resolution: e.g. sometimes 80 kVp will be better suited in that respect, although such voltage would increase the noise.

If higher radiation dose is used, an 'ultra-high resolution' scanning mode can give images with a spatial resolution of up to 24 line-pairs per centimeter and image slice width of 0.7 mm. In addition, a lower x-ray tube voltage can be used while maintaining good image quality due to the high dose.

Therefore, there is a practical problem due to the overall required high dose, to perform high definition scan protocols in cases of large tumor areas or tumors with multiple focal points or spread metastasizes, or even when it is not clear from a standard scan which region is the optimal candidate for Radiomics analysis.

The present offers a unique method to overcome these limitations.

WO2006090321 A1 may be considered to disclose a method for a medical image analysis, comprising the steps: a) Performing a first CT scan of a region of interest of a subject resulting in a first image with a first resolution; b) Applying a medical image processing method to the first image resulting in first values information representing a first analysis of the region of interest of the subject; c) Determining a range of interest of the subject based on the first information values; d) Performing a second CT scan of the range of interest of the subject resulting in a second image with a second resolution, wherein the second resolution is higher than the first resolution.

### SUMMARY OF THE DISCLOSURE

There may be a need to overcome the previously described disadvantages and/or limitation.

The object of the present invention is solved by the subject-matter of the independent claims, wherein further embodiments are incorporated in the dependent claims.

It should be noted that the following described aspects of the disclosure apply also for the system, the computer program element and the computer readable medium.

According to a first aspect of the present disclosure a method for a medical image analysis is provided, wherein said method comprising the steps:
a) Performing a first CT scan of a region of interest of a subject resulting in a first image with a first resolution;
b) Applying a medical image processing method to the first image resulting in first values representing a first analysis of the region of interest of the subject;
c) Determining a range of interest of the subject based on the first values;
d) Performing a second CT scan of the range of interest of the subject resulting in a second image with a second resolution, wherein the second resolution is higher than the first resolution; and
e) Applying the medical imaging processing method to the second image resulting in second values representing a second analysis of the range of interest of the subject.

As an effect, applying the same medical imaging processing method twice, namely in step b) and step e), the performance of the method may be substantially increased. The performance preferably relates to the time needed for performing the steps a) to e). In particular, processing units may be adapted for carrying out the medical imaging processing method, which reduces the processing time for each of steps b) and e).

As a further effect, the range of interest may be smaller, in particular significantly smaller than the region of interest. In particular, the range may be form or formed by a sub-region of the region of interest of the subject. Even though the second CT scan may be performed with a higher X-ray tube acceleration voltage than for the first CT scan, such that the second image comprises a higher resolution than the first image, a second X-ray dose resulting from the second scan may be smaller than a first X-ray dose resulting from the first CT scan. As an effect, the overall X-ray dose provided during both, the first and second CT scan may be limited to a minimum or may be small.

It to be understood that, although the terms "first" and "second" may be used herein to in combination with various features, these features should preferably not be limited by these respective terms. These terms may be only used to distinguish one feature from another. As used herein, the term "and/or" includes any and all combinations of one or more of the associated listed items.

In an example, an CT scan may also be referred to as a computed tomography scan.

In an example, the subject is a human subject.

In a further example, the region of interest of the subject may relate to a predefined region of interest of the subject.

In an example, X-ray radiation is projected on the subject during each of the first and second CT scan.

In an example, a image resulting from a CT scan may relate to or be a three dimensional image and/or a tomographical image.

In an example, the range of interest is or relates to a sub-region of the region of interest of the subject. As a result, the second scan may be limited to a sub-region of the region of interest of the subject. Thus, a dose of X-ray radiation projected to the subject, in particular during the second CT scan, may be reduced.

Preferably, the second resolution is higher than the first resolution by a factor of at least 1,5. As a result, the information provided by the second image may allow a more precise assessment of the range of interest of the subject.

In an example, the first CT scan may relate to a standard Computed Tomography scan. Therefore, and as a further example, in step a) standard Computed Tomography scan of a predefined region of interest of the subject may be performed. In a further example, the region of interest of the subject may relate to a relevant body region of the subject. In an even further example, the first CT scan may relate to a large coverage scan. Preferably, a large coverage scan may include a CT scan of at least a full organ area of the subject or of a full body area of the subject. Further preferred, during the first CT scan, the applied X-ray radiation dose may relate to a minimal X-ray radiation dose, in particular sufficient for the determination of a first image with a first resolution. This may then be sufficient for a subsequent clinical assessment of the subject, in particular with respect to the region of interest. As a result, the spatial resolution and low-contrast resolution of the first image may be not very high.

In step b), a medical image processing method is applied to the first image resulting in first values. In an example, the medical imaging processing method may also be referred to as a predefined medical image processing method, an imaging processing method or a predefined image processing method.

In an example, the step b) is automatically performed subsequently to step a).

In an example, the first analysis may also be referred to as a coarse analysis.

In an example, the medical image processing method may be configured to identify a section of the first image. The section preferably prelates to or shows a tumor of the subject. Further, the medical image processing method may be configured, based in the identified section, to determine the first values. The first values represent a first analysis of the region of interest of the subject, wherein the first analysis may relate to an identified tumor within the region of interest of the subject. Further, first values may indicate at least one sub-region of the region of interest, wherein each sub-region is formed by the identified tumor within the region of interest of the subject.

The medical image processing method is configured to identify a section of the first image by detecting a predefined pattern within the first image or at least one predefined feature within the first image. The predefined pattern or the at least one predefined feature may each be configured to represent an indication for a predefined disease, such as cancer. In an example, the at least one predefined feature may represent a predefined entropy and/or a predefined nun-uniformity. In a further example, the predefined features may relate to a Radiomic feature. Thus, the medical image processing method may be configured to detect a predefined pattern, which indicates a predefined disease, or a predefined feature, which indicates a predefined disease, and based on the respective detection result, the medical image processing method may be configured to determine first values, which represent a sub-region of the region of interest, where the predefined pattern or predefined feature has been detected.

In step c) a range of interest of the subject based on the first values is determined. The determination of the range of interest may be performed, such that the range of interest of the subject is formed by the at least one sub-region of the region of interest of the subject. As a result, the range of interest may represent the part of the region of interest, where at least one predefined pattern or at least one predefined feature is to be expected. It is therefore of advantage for a subsequent assessment, if the range of interest of the subject is scanned via a further CT scan, namely the second CT scan. As an effect, the second CT scan can be limited to the range of interest of the subject. As a result, a further projection of X-ray radiation to the subject may be limited, namely with respect to the geometrical dimensions and, in particular resulting therefrom, with respect to an X-ray radiation dose applied to the subject.

In an example, the step c) is automatically performed subsequently to step b).

In an example, the range of interest of the subject may also be referred to as an optimal limited scan range.

In step d), a second CT scan of the range of interest of the subject is performed.

In an example, the second CT scan may also be referred to as a Computed Tomography scan. The term "second" may be used for distinguishing purpose. Further preferred, the second CT scan is performed, such that the resulting second image comprises a higher resolution than the first image. Therefore, the second CT scan may also be referred to as a high-definition Computed Tomography scan. Optimization of other scan parameters may be done as well. As a result, a limited-range high-definition Computed Tomography scan may be performed.

In an example, the step d) is automatically performed subsequently to step c).

In step e), the medical image processing method is applied to the second image resulting in second values. The medical imaging processing method applied to the second image is the same medical image processing method as applied to the first image in step b). Preferably, reference is made in an analogous manner to the explanations, preferred embodiments, preferred examples, effects and/or advantages, which have been previously provided with respect to and/or in the context of the medical imaging processing method applied to the first image.

In an example, the step e) is automatically performed subsequently to step d).

In an example, the second analysis may also be referred to as a precise analysis or enhances analysis.

In an example, the medical image processing method may be configured to identify a section of the second image. The section preferably prelates to or shows a tumor of the subject. Further, the medical image processing method may be configured, based in the identified section, to determine the second values. The second values represent a second analysis of the range of interest of the subject, wherein the second analysis may relate to an identified tumor within the range of interest of the subject. Further, second values may indicate at least one sub-range of the range of interest, wherein each sub-range is formed by the identified tumor within the range of interest of the subject.

In an example, the medical image processing method may be configured to identify a section of the second image by detecting a predefined pattern within the second image or at least one predefined feature within the second image. The predefined pattern or the at least one predefined feature may each be configured to represent an indication for a predefined disease, such as cancer. In an example, the at least one predefined feature may represent a predefined entropy and/or a predefined nun-uniformity. In a further example, the predefined features may relate to a Radiomic feature. Thus, the medical image processing method may be configured to detect a predefined pattern, which indicates a predefined disease, or a predefined feature, which indicates a predefined disease, and based on the respective detection result, the medical image processing method may be configured to determine second values, which represent a sub-range of the range of interest of the subject, where the predefined pattern or predefined feature has been detected.

According to an exemplary embodiment of the method, the first CT scan is performed with a first X-ray dose and the second CT scan is performed with a second X-ray dose, wherein the second X-ray dose is higher than the first X-ray dose.

Preferably, the second X-ray dose is higher than the first X-ray dose by a factor of at least 1,5.

According to an exemplary embodiment of the method, the medical imaging processing method comprises at least one of the following sub-steps: Extracting of features of the first image; Classifying the extracted features; Determining an entropy of the region of interest of the subject; Determining an entropy of the range of interest of the subject; Determining a non-uniformity of the region of interest of the subject; and Determining a non-uniformity of the range of interest of the subject.

According to an exemplary embodiment of the method, the first values representing the first analysis comprise at least one value representing an extracted feature, at least one value representing a classified feature, at least one value representing a determined entropy and/or a at least one value representing determined non-uniformity.

According to an exemplary embodiment of the method, wherein the first values representing the first analysis comprise at least one value representing a sub-region of the region of interest, if a feature for the sub-region has been determined, and/or if the feature for the sub-region has been classified, in particular with respect to a predefined class or type, and/or if an entropy of at least a predefined entropy-value has been determined for the sub-region, and/or if a non-uniformity of at least a predefined degree of non-uniformity has been determined for said sub-region.

According to an exemplary embodiment of the method, the second values representing the second analysis comprise at least one value representing an extracted feature, at least one value representing a classified feature, at least one value representing a determined entropy and/or at least one value representing a determined non-uniformity.

According to an exemplary embodiment of the method, the first resolution refers to 8 to 10 line-pairs per centimeter (lp/cm) and/or wherein the second resolution refers to 11 to 24 line-pairs per centimeter (lp/cm).

According to an exemplary embodiment of the method, the medical image processing method applied in step b) to the first image comprises the sub-steps: Identifying at least one first lesion at the first image, wherein each first lesion represents a tumor; Determining a first periphery of each first lesion, wherein each first periphery represents an active region of the respective tumor; and Determining the first values based on the at least one first periphery.

According to an exemplary embodiment of the method, the medical image processing method applied in step e) to the second image comprises the sub-steps: Identifying at least one second lesion at the second image, wherein each second lesion represents a tumor; Determining a second periphery of each second lesion, wherein each second periphery represents an active region of the respective tumor; and Determining the second values based on the at least one second periphery.

According to an exemplary embodiment of the method, step c) comprises the sub-steps: Determining for each first lesion a sub-range within of region of interest of the subject, such that each sub-range represents the first periphery, or at least a part thereof, of the respective first lesion; and Determining the range of interest of the subject based on the at least on sub-range.

According to an exemplary embodiment of the method, at least one of the sub-ranges is an axial range, which represents an area of metastatic tissue, in particular a maximal area of metastatic tissue.

According to an exemplary embodiment of the method, the second CT scan is composed of several CT sub-scans, in particular at least one CT sub-scan for each sub-range.

According to an exemplary embodiment of the method, the second scan is a dynamic contrast enhanced CT scan.

According to an exemplary embodiment of the method, the method further comprises a segmentation step between step a) and b), wherein said segmentation step consists in manually or automatically segment a volume to be-analyzed in the scanned region of interest, the medical image processing method of step b) being applied only to said volume to-be analyzed.

According to an exemplary embodiment of the method, step b) comprises the following sub-steps: Extracting a plurality of volumetric slabs from the first image, and Calculating a significance metric of each slab, wherein the first values represent the significance metrics of the plurality of slabs; wherein step c) comprises the following sub-steps: Determining the slab of the plurality of slabs for which the highest significance metric has been calculated; and Determining the range of interest of the subject based on first values, such that the range of interest corresponds to at least a part of the slab for which the highest significance metric has been calculated.

In an example, a significance metric of a slab may indicate or represent a degree of a disease or a quantitative measure with respect to at least one predefined pattern of said slab and/or at least one feature of said slab. The at least one predefined pattern and/or the at least one predefined feature may be previously identified and/or detected in said slab.

According to a second aspect of the present disclosure, a system for a medical image analysis is provided. The system comprises a CT scanner; a control unit; and a processing unit; wherein the control unit is configured to control the CT scanner, such that the CT scanner performs a first CT scan of a region of interest of a subject resulting in a first image with a first resolution; wherein the processing unit is configured to apply a medical image processing method to the first image resulting in first values representing a first analysis of the region of interest of the subject; wherein the processing unit is configured to determine a range of interest of the subject based on the first values; wherein the control unit is configured to control the CT scanner, such that the CT scanner performs a second CT scan of the range of interest of the subject resulting in a second image with a second resolution, wherein the second resolution is higher than the first resolution; and wherein the processing unit is configured to apply the medical imaging processing method to the second image resulting in second values representing a second analysis of the range of interest of the subject.

It is understood that, without repeating here all the examples, features, effects and/or explanations provided with reference to the method according to the first aspect of the present disclosure, the system according to the second aspect of the present disclosure is preferably intended as being arranged and/or configured to carry out the above described method steps. Thus, all of the above provided examples, features, effects and/or explanations, although provided with reference to the method according to the first aspect of the present disclosure, are also to be preferably intended as being implemented by the system according to the second aspect of the present disclosure. This can be achieved, for example, by means of suitable hardware and/or software.

According to a third aspect of the present disclosure, a computer program element for controlling the system is provided, which, when being executed by a processing unit, is adapted to perform the method steps.

According to a fourth aspect of the present disclosure a computer readable medium having stored the program element is provided.

According to a fifth aspect of the present disclosure, a method for optimizing a medical image processing method is provided. The method according to the fifth aspect of the present disclosure comprises the steps:
a.1) Performing a Computed Tomography scan of a region of interest;
b.1) Applying the medical image processing method to the Computed Tomography scan to generate a coarse analysis of the scan;
c.1) Automatically designing and executing a limited range high-definition Computed Tomography scan based on the coarse analysis of the scan; and
d.1) Applying the medical imaging processing method to the limited range high-definition Computed Tomography scan.

It may be preferred that the preferred explanations, preferred examples, preferred features and/or effects previously provided with reference to the method according to the first aspect of the present disclosure may form in an analogous manner preferred explanations, preferred examples, preferred features and/or effects for the method according to the fifth aspect of the present disclosure.

At the first step a.1), a standard clinical Computed Tomography scan of a relevant body region is performed. For example, a large coverage scan that includes at least a full organ or body area. In this scan, the applied radiation dose is usually the minimal that is needed for the relevant clinical procedure of the specific patient. Consequently, the spatial resolution and low-contrast resolution are typically not very high.

At the second step b.1), a volume of interest may be determined, for example a tumor region which will be assessed. An algorithm may then generate volumetric mapping of a coarse analysis for this volume of interest. The coarse analysis preferably includes one or few primary mathematical features which are known to be significant for the anticipated disease. For example, in certain types of cancers the entropy or the non-uniformity may be the features that will be spatially mapped. This step may be done using known techniques. To measure the primary features coarsely, the standard clinical Computed Tomography scan is sufficient.

Based on the coarse analysis, an automatic algorithm may determine an optimal limited scan range for the high-definition Computed Tomography scan. Optimization of other scan parameters may be done as well. A limited-range high-definition Computed Tomography scan is then performed accordingly.

The fine high-quality medical image processing method may then be performed on the reconstructed high-definition Computed Tomography scan image volume. This fine analysis may include very large-number of mathematical features, and disease classification techniques.

The presentation of the analysis results to the user may be realized by several techniques.

According to an exemplary embodiment of the method according to the fifth aspect of the present disclosure, the medical imaging processing method is a Radiomics approach. However, other types of advanced medical analysis may benefit from the method according to the disclosure such as for example dynamic contrast-enhanced perfusion and permeability analysis, lung parenchyma analysis for COPD disease, or detailed plaque analysis in blood vessels.

According to an exemplary embodiment of the method according to the fifth aspect of the present disclosure, the Computed Tomography scan is preferably a low-dose scan. The meaning of 'low-dose' depends on the region which is to be imaged and, depending on the scanning protocol, it will be very clear to the one skilled in the art whether the scan is a 'low-dose' scan or not. Using a low-dose scan may hit the image resolution but it is not an issue as the purpose of the first scan is mainly to perform a coarse analysis in order to optimize the parameters of a high definition scan.

According to an exemplary embodiment of the method according to the fifth aspect of the present disclosure, the coarse analysis of the scan may comprise a spatial mapping of parameters, said parameters being preferably chosen among entropy and/or non-uniformity of the tissues or imaged tissues. As mentioned earlier, these parameters are especially relevant in certain kind of cancers as the tumor tissue typically have a different entropy or non-uniformity than healthy tissues. As an option, other parameters may be selected for the coarse analysis such as simple parameters based on the local mean HU intensity or the standard deviation.

According to an exemplary embodiment of the method according to the fifth aspect of the present disclosure, the limited range high definition Computed Tomography scan is preferably a high dose scan. Indeed, a higher dose allows for a higher resolution. What is preferably meant by 'high dose scan' is a scan which irradiates at least a part of the imaged region with a high dose. It does not have to irradiate the whole imaged zone with a high dose. Indeed, such high-dose scan typically targets only a small region of high interest with a higher dose while the vast majority of the imaged zone undergoes only a low irradiation dose. This allows to get a high quality on the regions which are of interest without submitting the patient to an overall important dose. It is allowed by the previously performed optimization of the scan parameters.

According to an exemplary embodiment of the method according to the fifth aspect of the present disclosure, the limited range high definition Computed Tomography scan has a high spatial resolution and/or high low-contrast resolution.

According to an exemplary embodiment of the method according to the fifth aspect of the present disclosure, the limited range high definition Computed Tomography scan may have a high spatial resolution and/or low-contrast resolution. Both are of interest.

According to an exemplary embodiment of the method according to the fifth aspect of the present disclosure, the limited range of the limited range high-definition Computed Tomography scan may comprise the periphery of a lesion, said periphery of a lesion being preferably an active region of a tumor. Indeed, the center of a tumor can be necrotic or, at least, not the most active and aggressive part of the tumor. In such particular case, the center is not the part which needs the highest resolution.

According to an exemplary embodiment of the method according to the fifth aspect of the present disclosure, the limited range of the limited range high-definition Computed Tomography scan is preferably an axial range which contains the maximal area of metastatic tissue.

According to an exemplary embodiment of the method according to the fifth aspect of the present disclosure, the limited range high-definition Computed Tomography scan may be composed of several sub-scans. In some situations, several narrow scan may be an optimal alternative as a single scan, especially when there is no utterly satisfying axial range.

According to an exemplary embodiment of the method according to the fifth aspect of the present disclosure, the limited range high-definition Computed Tomography scan may be a multi-phasic scan.

According to an exemplary embodiment of the method according to the fifth aspect of the present disclosure, the limited range high-definition Computed Tomography scan may be a dynamic contrast enhanced scan.

According to an exemplary embodiment of the method according to the fifth aspect of the present disclosure, the method according to the fifth aspect of the present disclosure may further comprise a segmentation step between step a.1) and b.1), wherein said segmentation step consists in manually or automatically segment a volume to be-analyzed in the scanned region of interest, the medical image processing method of step b.1) being applied only to said volume to-be analyzed. If the segmentation is done automatically, it may for example be done by using CAD algorithms for specific known disease or organ. In another option, the whole imaged volume may be selected for the next step, without needing any segmentation.

According to an exemplary embodiment of the method according to the fifth aspect of the present disclosure, designing the limited range high-definition Computed Tomography scan comprises:
a.2) Extracting a volumetric slab from the Computed Tomography scan,
b.2) Calculating a global significance metric of the slab based on the coarse analysis of the scan,
c.2) Repeating steps a and b a predetermined number of time for different volumetric slabs of the Computed Tomography scan, and
d.2) Determining the slab which has the highest global significance metric and setting the limited range of the limited range high-definition Computed Tomography scan to a corresponding position.

According to an exemplary embodiment of the method according to the fifth aspect of the present disclosure, the designing step of the limited range high-definition Computed Tomography scan may comprise: Extracting a volumetric slab from the Computed Tomography scan, Calculating a global significance metric of the slab based on the coarse analysis of the scan, Repeating steps a and b a predetermined number of time for different volumetric slabs of the Computed Tomography scan, and Determining the slab which has the highest global significance metric and setting the limited range of the limited range high-definition Computed Tomography scan to a corresponding optimal position.

According to an exemplary embodiment of the method according to the fifth aspect of the present disclosure, the initial slab can be determined with relation to the initial limited axial range width, and located in a first determined position along the patient body scan. For example on one side of the initially segmented region. In case the medical image processing method consists in a Radiomics approach, the significant metric may be based on the group of local coarse Radiomics analysis values. For example, the metric may be the sum of the values above a pre-determined threshold. In the end, the algorithm may perform a fine adjustment of the determined axial range and the high-definition scan parameters based on the result of the previous steps and on predetermined limitation rules, e.g. to further limiting the axial range if the included data for the Radiomics analysis is still sufficient. Said limitation rules may be constant or tailored for the specific patient or protocol. For example, the rules may relate to the maximal allowed axial coverage, the number of multiple narrow-range scans, the maximal allowed total radiation dose, or dose modulation schemes.

It may be preferred that the preferred explanations, preferred examples, preferred features and/or effects previously provided with reference to the method according to the fifth aspect of the present disclosure may form in an analogous manner preferred explanations, preferred examples, preferred features and/or effects for the method according to the first aspect of the present disclosure.

According to a sixth aspect of the present disclosure, a device configured to implement a method according to the fifth aspect of the present disclosure is provided, comprising a Computed Tomography scanner controlled by a processor configured to apply the medical image processing method.

According to a seventh aspect of the present disclosure, a computer readable storage medium encoded with computer readable instructions is provided, which, when executed by a processor, causes the processor to perform a method according to the disclosure.

### BRIEF DESCRIPTION OF THE FIGURES

The invention shall be better understood by reading the following detailed description of an embodiment of the invention and by examining the annexed drawing, on which:
FIGURE 1 is a typical Computed Tomography scanner,
FIGURE 2 schematically illustrates different possible axial ranges preferably for performing a high-definition scan in accordance with the invention,
FIGURE 3 is a general flowchart of a method in accordance with the invention,
FIGURE 4 is a detailed flowchart, in particular of the technique for determining the limited-range high-definition scan protocol, preferably of the method of FIGURE 3,
FIGURE 5 shows the results of one possible technique to obtain the volumetric mapping of coarse Radiomics analysis, and
FIGURE 6 schematically illustrates an embodiment of the method in accordance with the present invention.

The drawings are only for the purpose of illustrating preferred embodiments and are not to be construed as limiting the invention. To better visualize certain features may be omitted or dimensions may be not be according to scale.

### DETAILED DESCRIPTION OF EMBODIMENTS

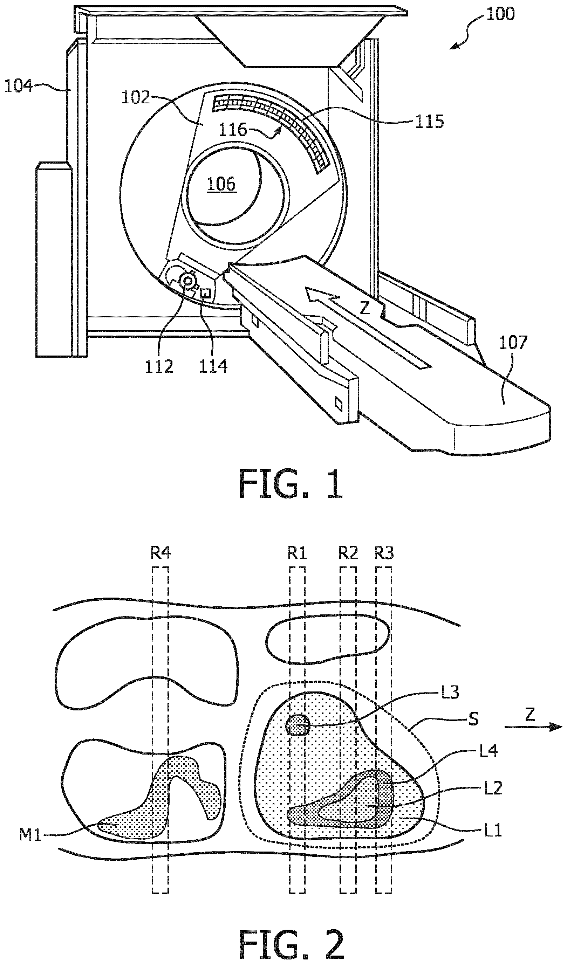

FIGURE 1 schematically illustrates an example imaging system 100, such as a computed tomography (CT) scanner. The imaging system 100 includes a rotating gantry 102 and a stationary gantry 104. The rotating gantry 102 is rotatably supported by the stationary gantry 104. The rotating gantry 102 is configured to rotate around an examination region 106 about a longitudinal or z-axis. The imaging system 100 further includes a subject support 107 that supports a subject or object in the examination region 106 before, during and/or after scanning. The subject support 107 can also be used to load and/or unload the subject or object into or from the examination region 106. The imaging system 100 further includes a radiation source 112, such as an x-ray tube, that is rotatably supported by the rotating gantry 102. The radiation source 112 rotates with the rotating gantry 102 around the examination region 106 and is configured to generate and emit radiation that traverses the examination region 106. The imaging system 100 further includes a radiation source controller 114. The radiation source controller 114 is configured to modulate a flux of the generated radiation. For example, the radiation controller 114 can selectively change a cathode heating current of the radiation source 112, apply a charge to inhibit electron flow of the radiation source 112, filter the emitted radiation, etc. to modulate the flux. In the illustrated example, the radiation source controller 114 modulates the flux based on a predetermined modulation pattern.

The imaging system 100 further includes a one or two dimensional array 115 of radiation sensitive detector pixels 116. The pixels 116 are located opposite the radiation source 112, across the examination region 106, detect radiation traversing the examination region 106, and generate an electrical signal (projection data) indicative thereof.

In the illustrated embodiment of the invention, the medical image processing method will be a Radiomics approach. An aspect, in particular a key aspect, of the invention may be the designing of a high-definition CT scan protocol dedicated for the specific imaged patient, where the scan may include a limited but optimized coverage or exposure range; this may be crucial in order to both limiting the applied radiation dose and to obtain the most important data for the Radiomics analysis. The optimal high definition scan range in the context of CT oncology applications may not be necessarily on the center of a large tumor since the center may be necrotic or inactive, while the most active or aggressive tumor region may be more on the lesion peripheries. In addition, the optimal scanning axial range may be this which contains the maximal area with several small metastasizes. In some situations, several narrow scans may give an optimal compromise.

It is also important to emphasize that it may be much more important in Radiomics analysis (for capturing the aggressiveness phenotype of tumor) to analyze especially the most significant regions and not to average them with adjacent inactive or normal tissue regions.

While considering all these aspects, the optimized limited range may be an advantageous approach.

Additional option or extension is that the limited high definition scan may be a multi-phasic scan or a dynamic contrast enhanced scan, while other axial ranges are not exposed to the excess radiation.

In FIGURE 2, a standard body Computed Tomography scan covers several organs. In the example, a relevant organ, in particular for Radiomics analysis, e.g. liver, is roughly segmented, shown with the contour 'S'. A volumetric mapping of a coarse Radiomics analysis is performed on the selected volume. The illustration shows several mapping values which indicate possible significant features, for example that may indicate or be correlated with tumor aggressiveness. 'L1' is a background value which show no significance. 'L2' is a low significance value, for example related to necrotic region in a tumor. 'L3' is a medium significance value which may relate to initial tumor metastasis. 'L4' is a high significance value which may relate to aggressive tumor tissue mainly on the tumor boundaries.

R1, R2, and R3 indicate for example three possible limited axial ranges for a high-definition scan. By applying the proposed analysis it can be found that overall it is more optimized for fine Radiomics analysis to select and scan range R1 or R3, but range R2 can be neglected since most of the coarse Radiomics mapping values are less significant in this slab.

In another organ, e.g. lung, the region with values 'M1' indicates distributed disease relative to a certain feature, in particular to a certain Radiomics feature. The selected limited axial range optimally covers the most of the pixels with the high significance values.

Of course, it is possible that several separated or connected limited axial-ranges will be selected and scanned, in particular for the Radiomics analysis.

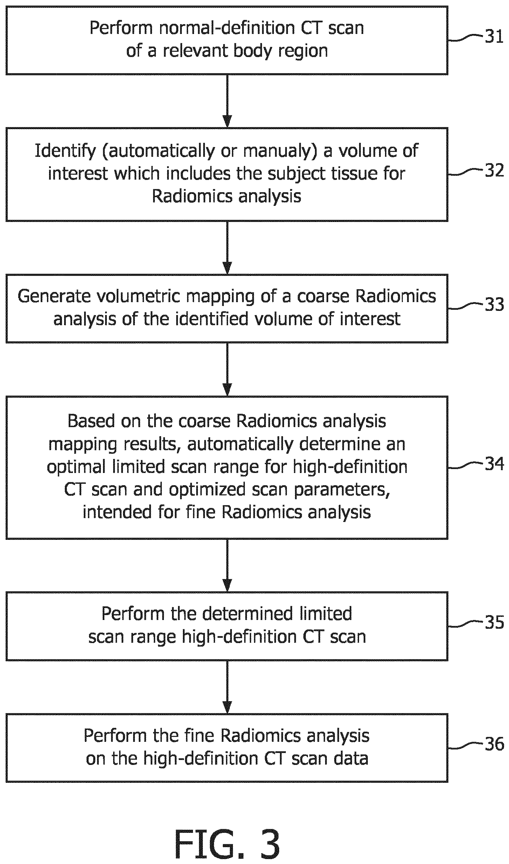

FIGURE 3 illustrates the main flowchart of the exemplified method. In the first step 31, a normal-definition CT scan of a relevant body region is performed. Usually this is a large coverage scan that covers at least a full organ or area such as head, chest, abdomen or the full body. In this scan, the applied radiation dose is usually the minimal that is needed for the relevant standard clinical diagnostic of the specific patient. The CT scan may be performed with or without contrast agent administration, as required.

In step 32, after the images are reconstructed from the normal-definition CT scan, an automatic or manual procedure identifies a volume of interest which includes the subject tissue for Radiomics analysis. In a manual option the user may scroll the screen through the image volume and select with a proper user-interface tool the relevant region (or several regions) of interest, for example by roughly contouring a notable tumor area. The selection may be done automatically for example by using CAD algorithms for specific known disease. In another option, the whole imaged volume may be selected for the next step, without needing any segmentation.

In step 33, after the volume of interest was determined, an algorithm generates volumetric mapping of a coarse Radiomics analysis for this volume of interest. This coarse analysis includes one or few features which are known to be significant for the anticipated disease. For example, in certain types of cancers the entropy, non-uniformity, or relative contrast agent enhancement may be the main features which will be mapped. Note that one of the advantages of the volumetric mapping is that no tissue segmentation is needed in this step.

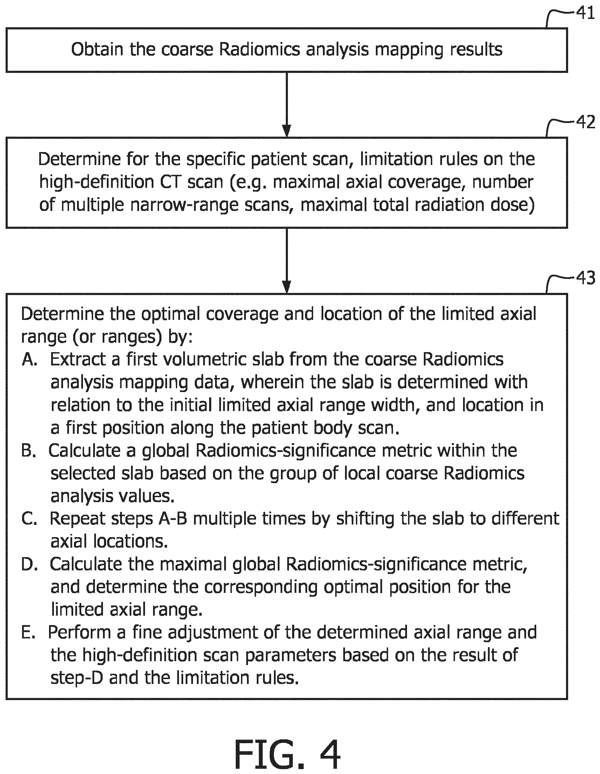

In step 34, after the coarse Radiomics analysis mapping results was calculated, an automatic algorithm determines, based on the coarse mapping, an optimal limited scan range for a high-definition CT scan which is intended for the required fine Radiomics analysis. Optimization of other scan parameters may be done as well. The details of this algorithm are described in FIGURE 4.

In step 35, the determined limited-range high-definition CT scan is performed. The scan may include one or several limited axial ranges. For example, the scan range may covers few centimeters along the Z direction, placed on a portion of a tumor. The high-definition CT scan will usually set with relatively high radiation dose for that region only, in order to achieve superior image quality properties relative to the normal-definition CT scan of that region.

Eventually, after the high-definition images are reconstructed, the fine Radiomics analysis is performed on the high-definition CT scan data. This fine analysis may include large-number of mathematical features, and disease classification techniques as described in the references. The high-definition CT scan may include remaining contrast agent in the body from the previous scan (i.e. late phase), may include a new contrast agent administration, or may be a non-contrasted scan.

FIGURE 4 details step 4 of the method represented in FIGURE 3. In step 41, the coarse Radiomics analysis mapping results is obtained. The mapping is a volume of voxels, each has one or more values corresponding to the analyzed mathematical features, i.e. there can be several different maps for the same spatial volume (such as local entropy map, local non-uniformity map, or contrast agent enhancement distribution relative to a given criteria).

In step 42, in order to calculate the optimal high-definition scan parameters, the algorithm should have as an input, a set of limitation rules on the high-definition scan (that may be constant or tailored for the specific patient or protocol). For example, the rules may relate to the maximal allowed axial coverage, the number of multiple narrow-range scans, the maximal allowed total radiation dose, or dose modulation schemes.

Eventually, at step 43, the algorithm determines the optimal coverage and location of the limited axial range (or ranges) by:
A. The algorithm extracts a first volumetric slab from the coarse Radiomics analysis mapping data, wherein the slab is determined with relation to the initial limited axial range width, and located in a first determined position along the patient body scan. For example on one side of the initially segmented region.
B. The algorithm calculates a global Radiomics-significance metric within the selected slab based on the group of local coarse Radiomics analysis values. For example, the metric may be the sum of the values, above a pre-determined threshold.
C. The algorithm repeats steps A-B multiple times by shifting the slab to different axial positions.
D. The algorithm calculates the maximal global Radiomics-significance metric from the group of values calculated in the previous step, and determines the corresponding optimal position for the limited axial range.
E. The algorithm performs a fine adjustment of the determined axial range and the high-definition scan parameters based on the result of the previous step and the limitation rules, e.g. to further limiting the axial range if the included data for the Radiomics analysis is still sufficient.

In the proposed method, a first scan is used to plan the scan positions for the second successive scan. In such scenario some inaccuracies may occur in principle, due to sporadic patient motion between the two scans. To overcome such a problem, a verification step may be applied before the high-definition scan. In the verification procedure a fast low-dose CT scan of the determined region (of the high definition scan) can be performed and used just to validate the correct position. The validation can be done automatically by image registration with the first scan. If needed, an automatic axial position correction can be performed for the planning of the high definition scan.

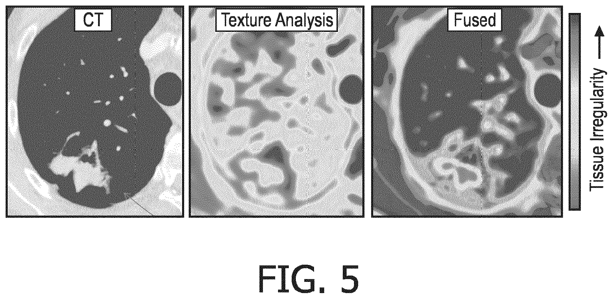

FIGURE 5 shows one possible technique to obtain the volumetric mapping of coarse Radiomics analysis. Here the mapping values correspond to one selected texture feature, which is one mathematical form of calculating entropy of co-occurrence matrix. For final visualization and result presentation, the high-definition analysis can be viewed with (e.g. fused) the coarse analysis results or with the conventional CT images. Some intermediate steps can be visualized as well.

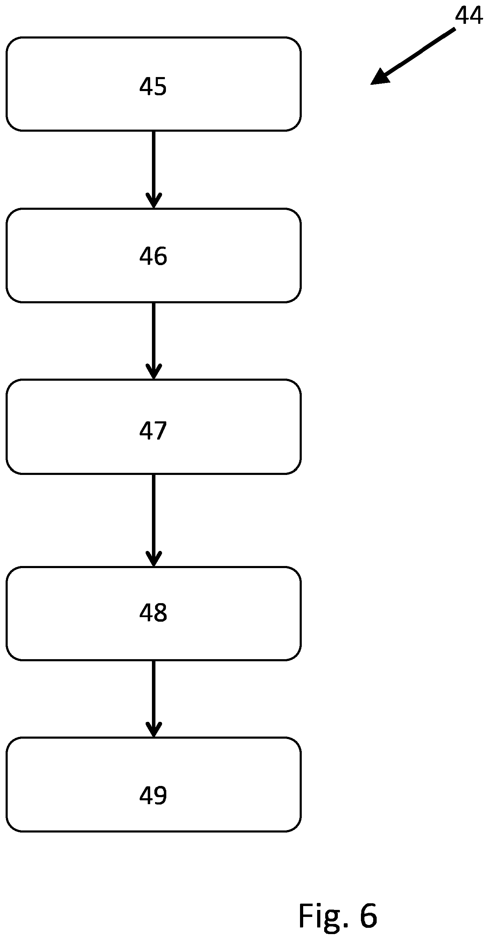

Figure 6 schematically shows an example of a further example of a method 44 in accordance with the present invention. The method 44 comprises the following:
In a first step 45, a first CT scan of a region of interest of a subject is performed resulting in a first image with a first resolution.

In a second step 46, a medical image processing method is applied to the first image resulting in first values representing a first analysis of the region of interest of the subject.

In a third step 47, a range of interest of the subject is determined based on the first values.

In a fifth step 48, a second CT scan of the range of interest of the subject is performed resulting in a second image with a second resolution, wherein the second resolution is higher than the first resolution.

In a sixth step 49, the medical imaging processing method is applied to the second image resulting in second values representing a second analysis of the range of interest of the subject.

It has to be noted that embodiments of the invention are described with reference to different subject matters. In particular, some embodiments are described with reference to a method according to the fifth aspect of the present invention whereas other embodiments are described with reference to a method according to the first aspect of the present invention.

While the invention has been illustrated and described in detail in the drawings and foregoing description, such illustration and description are to be considered illustrative or exemplary and not restrictive; the invention is not limited to the discussed embodiments. The invention is not limited to the disclosed embodiments. Other variations to the disclosed embodiments may be understood and effected by those skilled in the art in practicing a claimed invention, from a study of the drawings, the disclosure, and the dependent claims.

For example, the proposed concept of the localized high-definition analysis may be used to accurately guide real biopsy sampling. As a matter of fact, one problem with real biopsy sampling is that only small part of the tumor volume can be analyzed. Since in many progressive tumors there is large tissue variability between different locations, it may be important to take samples (by interventional procedure) especially from those regions which show high significance in the Radiomics analysis.

Other variations to the disclosed embodiments may be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A processing unit, single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. Method for a medical image analysis, comprising the steps:
a) Performing a first CT scan of a region of interest of a subject resulting in a first image with a first resolution;
b) Applying a medical image processing method to the first image resulting in first values representing a first analysis of the region of interest of the subject, wherein the medical image processing method is configured to identify a section of the first image by detecting a predefined pattern or at least one predefined feature within the first image;
c) Determining a range of interest of the subject based on the first values, wherein the range of interest represents a part of the region of interest where at least one predefined pattern or predefined feature is to be expected;
d) Performing a second CT scan of the range of interest of the subject resulting in a second image with a second resolution, wherein the second resolution is higher than the first resolution; and
e) Applying the same medical imaging processing method to the second image resulting in second values representing a second analysis of the range of interest of the subject, wherein the medical imaging processing method is configured to identify a section of the second image by detecting a predefined pattern or at least one predefined featured within the second image.

2. Method according to any of the preceding claims, wherein the first CT scan is performed with a first X-ray dose and the second CT scan is performed with a second X-ray dose, wherein the second X-ray dose is higher than the first X-ray dose.

3. Method according to the preceding claim, wherein the medical imaging processing method comprises at least one of the following sub-steps:
- Extracting of features of the first image;
- Classifying the extracted features;
- Determining an entropy of the region of interest of the subject;
- Determining an entropy of the range of interest of the subject;
- Determining a non-uniformity of the region of interest of the subject; and
- Determining a non-uniformity of the range of interest of the subject.

4. Method according to the preceding claim, wherein the first values representing the first analysis comprise at least one value representing an extracted feature, at least one value representing a classified feature, at least one value representing a determined entropy and/or a at least one value representing determined non-uniformity.

5. Method according to any of the preceding claims 3 or 4, wherein the first values representing the first analysis comprise at least one value representing a sub-region of the region of interest,
if a feature for the sub-region has been determined,
and/or if the feature for the sub-region has been classified, in particular with respect to a predefined class or type,
and/or if an entropy of at least a predefined entropy-value has been determined for the sub-region,
and/or if a non-uniformity of at least a predefined degree of non-uniformity has been determined for said sub-region.

6. Method according to any of the preceding claims 3 to 5, wherein the second values representing the second analysis comprise at least one value representing an extracted feature, at least one value representing a classified feature, at least one value representing a determined entropy and/or at least one value representing a determined non-uniformity.

7. Method according to any of the preceding claims, wherein the first resolution refers to 8 to 10 line-pairs per centimeter (lp/cm) and/or wherein the second resolution refers to 11 to 24 line-pairs per centimeter (lp/cm).

8. Method according to any of the preceding claims, wherein the medical image processing method applied in step b) to the first image comprises the sub-steps:
- Identifying at least one first lesion at the first image, wherein each first lesion represents a tumor;
- Determining a first periphery of each first lesion, wherein each first periphery represents an active region of the respective tumor; and
- Determining the first values based on the at least one first periphery.

9. Method according to any of the preceding claims, wherein the medical image processing method applied in step e) to the second image comprises the sub-steps:
- Identifying at least one second lesion at the second image, wherein each second lesion represents a tumor;
- Determining a second periphery of each second lesion, wherein each second periphery represents an active region of the respective tumor; and
- Determining the second values based on the at least one second periphery.

10. Method according to the preceding claim, wherein step c) comprises the sub-steps:
- Determining for each first lesion a sub-range within of region of interest of the subject, such that each sub-range represents the first periphery, or at least a part thereof, of the respective first lesion; and
- Determining the range of interest of the subject based on the at least on sub-range.

11. Method according to the preceding claim, wherein at least one of the sub-ranges is an axial range, which represents an area of metastatic tissue, in particular a maximal area of metastatic tissue.

12. Method according to any of the preceding claims, wherein the second CT scan is composed of several CT sub-scans, in particular at least one CT sub-scan for each sub-range.

13. Method according to any of the preceding claims, wherein the second scan is a dynamic contrast enhanced CT scan.

14. Method according to any of the preceding claims, further comprising a segmentation step between step a) and b), wherein said segmentation step consists in manually or automatically segment a volume to be-analyzed in the scanned region of interest, the medical image processing method of step b) being applied only to said volume to-be analyzed.

15. Method according to any of the preceding claims,
wherein step b) comprises the following sub-steps:
- Extracting a plurality of volumetric slabs from the first image, and
- Calculating a significance metric of each slab, wherein the first values represent the significance metrics of the plurality of slabs;
wherein step c) comprises the following sub-steps:
- Determining the slab of the plurality of slabs for which the highest significance metric has been calculated; and
- Determining the range of interest of the subject based on first values, such that the range of interest corresponds to at least a part of the slab for which the highest significance metric has been calculated.

16. System for a medical image analysis, comprising:
- a CT scanner;
- a control unit; and
- a processing unit;
wherein the control unit is configured to control the CT scanner, such that the CT scanner performs a first CT scan of a region of interest of a subject resulting in a first image with a first resolution;
wherein the processing unit is configured to apply a medical image processing method to the first image resulting in first values representing a first analysis of the region of interest of the subject, wherein the medical image processing method is configured to identify a section of the first image by detecting a predefined pattern or at least one predefined feature within the first image;
wherein the processing unit is configured to determine a range of interest of the subject based on the first values wherein the range of interest represents a part of the region of interest where at least one predefined pattern or predefined feature is to be expected;
wherein the control unit is configured to control the CT scanner, such that the CT scanner performs a second CT scan of the range of interest of the subject resulting in a second image with a second resolution, wherein the second resolution is higher than the first resolution; and
wherein the processing unit is configured to apply the same medical imaging processing method to the second image resulting in second values representing a second analysis of the range of interest of the subject, wherein the medical imaging processing method is configured to identify a section of the second image by detecting a predefined pattern or at least one predefined featured within the second image.

17. A computer program element for controlling the system according to claims 16, which, when being executed by a processing unit, is adapted to perform the method steps of any of the claims 1 to 15.

18. A computer readable medium having stored the program element of claim 17.

## Patentansprüche

1. Verfahren für eine medizinische Bildanalyse, umfassend die Schritte von:
a) Durchführen eines ersten CT-Scans einer interessierenden Region eines Subjekts, was zu einem ersten Bild mit einer ersten Auflösung führt;
b) Anwenden eines medizinischen Bildverarbeitungsverfahrens auf das erste Bild, was zu ersten Werten führt, die eine erste Analyse der interessierenden Region des Subjekts darstellen, wobei das medizinische Bildverarbeitungsverfahren konfiguriert ist, um einen Abschnitt des ersten Bildes durch Erfassen eines vordefinierten Musters oder mindestens ein vordefiniertes Merkmal innerhalb des ersten Bildes zu identifizieren;
c) Bestimmen eines interessierenden Bereichs des Subjekts basierend auf den ersten Werten, wobei der interessierende Bereich einen Teil der interessierenden Region darstellt, in dem mindestens ein vordefiniertes Muster oder vordefiniertes Merkmal zu erwarten ist;
d) Durchführen eines zweiten CT-Scans des interessierenden Bereichs des Subjekts, was zu einem zweiten Bild mit einer zweiten Auflösung führt, wobei die zweite Auflösung höher als die erste Auflösung ist; und
e) Anwenden des gleichen medizinischen Bildverarbeitungsverfahrens auf das zweite Bild, was zu zweiten Werten führt, die eine zweite Analyse des interessierenden Bereichs des Subjekts darstellen, wobei das medizinische Bildverarbeitungsverfahren konfiguriert ist, um einen Abschnitt des zweiten Bildes durch Erfassen eines vordefinierten Musters oder mindestens eines vordefinierten Merkmals im zweiten Bild zu identifizieren.

2. Verfahren nach einem der vorhergehenden Ansprüche, wobei der erste CT-Scan mit einer ersten Röntgendosis und der zweite CT-Scan mit einer zweiten Röntgendosis durchgeführt wird, wobei die zweite Röntgendosis höher als die erste Röntgendosis ist.

3. Verfahren nach dem vorhergehenden Anspruch, wobei das medizinische Bildverarbeitungsverfahren mindestens einen der folgenden Teilschritte umfasst:
- Extrahieren von Merkmalen des ersten Bildes;
- Klassifizierung der extrahierten Merkmale;
- Bestimmen einer Entropie der interessierenden Region des Subjekts;
- Bestimmen einer Entropie des interessierenden Bereichs des Subjekts;
- Bestimmen einer Ungleichmäßigkeit der interessierenden Region des Subjekts; und
- Bestimmen einer Ungleichmäßigkeit des interessierenden Bereichs des Subjekts.

4. Verfahren nach dem vorhergehenden Anspruch, wobei die ersten Werte, die die erste Analyse darstellen, mindestens einen Wert umfassen, der ein extrahiertes Merkmal darstellt, mindestens einen Wert, der ein klassifiziertes Merkmal darstellt, mindestens einen Wert, der eine bestimmte Entropie darstellt, und/oder mindestens ein Wert, der eine bestimmte Ungleichmäßigkeit darstellt.

5. Verfahren nach einem der vorhergehenden Ansprüche 3 oder 4, wobei die ersten Werte, die die erste Analyse darstellen, mindestens einen Wert umfassen, der eine Unterregion der interessierenden Region darstellt,
wenn ein Merkmal für die Unterregion bestimmt wurde,
und/oder wenn das Merkmal für die Unterregion klassifiziert wurde, insbesondere in Bezug auf eine vordefinierte Klasse oder einen vordefinierten Typ,
und/oder wenn eine Entropie von mindestens einem vordefinierten Entropiewert für die Unterregion bestimmt wurde,
und/oder wenn für den Teilbereich eine Ungleichmäßigkeit von mindestens einem vordefinierten Grad an Ungleichmäßigkeit bestimmt wurde.

6. Verfahren nach einem der vorhergehenden Ansprüche 3 bis 5, wobei die zweiten Werte, die die zweite Analyse darstellen, mindestens einen Wert umfassen, der ein extrahiertes Merkmal darstellt, mindestens einen Wert, der ein klassifiziertes Merkmal darstellt, mindestens einen Wert, der eine bestimmte Entropie darstellt, und/oder mindestens ein Wert, der eine bestimmte Ungleichmäßigkeit darstellt.

7. Verfahren nach einem der vorhergehenden Ansprüche, wobei sich die erste Auflösung auf 8 bis 10 Linienpaare pro Zentimeter (lp/cm) bezieht, und/oder sich die zweite Auflösung auf 11 bis 24 Linienpaare pro Zentimeter (lp/cm) bezieht.

8. Verfahren nach einem der vorhergehenden Ansprüche, wobei das in Schritt b) auf das erste Bild angewandte medizinische Bildverarbeitungsverfahren die Unterschritte umfasst von:
- Identifizieren mindestens einer ersten Läsion am ersten Bild, wobei jede erste Läsion einen Tumor darstellt;
- Bestimmen einer ersten Peripherie jeder ersten Läsion, wobei jede erste Peripherie eine aktive Region des jeweiligen Tumors darstellt; und
- Bestimmen der ersten Werte basierend auf der mindestens einen ersten Peripherie.

9. Verfahren nach einem der vorhergehenden Ansprüche, wobei das in Schritt e) auf das zweite Bild angewandte medizinische Bildverarbeitungsverfahren die Unterschritte umfasst von:
- Identifizieren von mindestens einer zweiten Läsion im zweiten Bild, wobei jede zweite Läsion einen Tumor darstellt;
- Bestimmen einer zweiten Peripherie jeder zweiten Läsion, wobei jede zweite Peripherie eine aktive Region des jeweiligen Tumors darstellt; und
- Bestimmen der zweiten Werte basierend auf der mindestens einen zweiten Peripherie.

10. Verfahren nach dem vorhergehenden Anspruch, wobei der Schritt c) die Teilschritte umfasst von:
- Bestimmen einer Unterregion innerhalb der interessierenden Region des Subjekts für jede erste Läsion, so dass jede Unterregion die erste Peripherie oder zumindest einen Teil davon der jeweiligen ersten Läsion darstellt; und
- Bestimmen des interessierenden Bereichs des Subjekts basierend auf dem mindestens einen Teilbereich.

11. Verfahren nach dem vorhergehenden Anspruch, wobei mindestens einer der Teilbereiche ein axialer Bereich ist, der einen Bereich von metastasiertem Gewebe darstellt, insbesondere einen maximalen Bereich von metastasiertem Gewebe.

12. Verfahren nach einem der vorhergehenden Ansprüche, wobei der zweite CT-Scan aus mehreren CT-Unterscans besteht, insbesondere mindestens einem CT-Unterscan für jeden Teilbereich.

13. Verfahren nach einem der vorhergehenden Ansprüche, wobei der zweite Scan ein dynamischer kontrastverstärkter CT-Scan ist.

14. Verfahren nach einem der vorhergehenden Ansprüche, ferner umfassend einen Segmentierungsschritt zwischen Schritt a) und b), wobei der Segmentierungsschritt darin besteht, ein zu analysierendes Volumen in der abgetasteten interessierenden Region, manuell oder automatisch zu segmentieren, wobei das medizinische Bildverarbeitungsverfahren von Schritt b) nur auf das zu analysierende Volumen angewendet wird.

15. Verfahren nach einem der vorhergehenden Ansprüche,
wobei der Schritt b) die folgenden Teilschritte umfasst:
- Extrahieren mehrerer volumetrischer Platten aus dem ersten Bild, und
- Berechnen einer Signifikanzmetrik jeder Platte, wobei die ersten Werte die Signifikanzmetriken der Vielzahl von Platten darstellen;
wobei der Schritt c) die folgenden Teilschritte umfasst:
- Bestimmen der Platte aus mehreren Platten, für die die Metrik mit der höchsten Signifikanz berechnet wurde; und
- Bestimmen des interessierenden Bereichs des Subjekts anhand der ersten Werte, sodass der interessierende Bereich mindestens einem Teil der Platte entspricht, für den die Metrik mit der höchsten Signifikanz berechnet wurde.

16. System für eine medizinische Bildanalyse, umfassend:
- ein CT-Scanner;
- eine Steuereinheit; und
- eine Verarbeitungseinheit;
wobei die Steuereinheit konfiguriert ist, um den CT-Scanner so zu steuern, dass der CT-Scanner einen ersten CT-Scan einer interessierenden Region eines Subjekts durchführt, was zu einem ersten Bild mit einer ersten Auflösung führt;
wobei die Verarbeitungseinheit konfiguriert ist, um ein medizinisches Bildverarbeitungsverfahren auf das erste Bild anzuwenden, was zu ersten Werten führt, die eine erste Analyse der interessierenden Region des Subjekts darstellen, wobei das medizinische Bildverarbeitungsverfahren konfiguriert ist, um einen Abschnitt des ersten Bildes durch Erfassen eines vordefinierten Musters oder mindestens eines vordefinierten Merkmals innerhalb des ersten Bildes zu identifizieren;
wobei die Verarbeitungseinheit konfiguriert ist, um einen interessierenden Bereich des Subjekts basierend auf den ersten Werten zu bestimmen, wobei der interessierende Bereich einen Teil der interessierenden Region darstellt, in dem mindestens ein vordefiniertes Muster oder vordefiniertes Merkmal zu erwarten ist;
wobei die Steuereinheit konfiguriert ist, um den CT-Scanner so zu steuern, dass der CT-Scanner einen zweiten CT-Scan des interessierenden Bereichs des Subjekts durchführt, was zu einem zweiten Bild mit einer zweiten Auflösung führt, wobei die zweite Auflösung höher als die erste Auflösung ist; und
wobei die Verarbeitungseinheit konfiguriert ist, um das gleiche medizinische Bildverarbeitungsverfahren auf das zweite Bild anzuwenden, was zu zweiten Werten führt, die eine zweite Analyse des interessierenden Bereichs des Subjekts darstellen, wobei das medizinische Bildverarbeitungsverfahren konfiguriert ist, um einen Abschnitt des zweiten Bildes durch Erfassen eines vordefinierten Musters oder mindestens eines vordefinierten Merkmals im zweiten Bild zu identifizieren.

17. Computerprogrammelement zum Steuern des Systems nach Anspruch 16, das, wenn es von einer Verarbeitungseinheit ausgeführt wird, angepasst ist, um die Verfahrensschritte eines der Ansprüche 1 bis 15 auszuführen.

18. Computerlesbares Medium, das das Programmelement nach Anspruch 17 gespeichert hat.

## Revendications

1. Procédé d'analyse d'image médicale, comprenant les étapes:
a) Réalisation d'un premier balayage de tomodensitométrie d'une région d'intérêt d'un sujet résultant en une première image avec une première résolution;
b) Application d'un procédé de traitement d'image médicale à la première image résultant en des premières valeurs représentant une première analyse de la région d'intérêt du sujet, où le procédé de traitement d'image médicale est configuré pour identifier une section de la première image en détectant un motif prédéfini ou au moins une caractéristique prédéfinie dans la première image;
c) Détermination d'une plage d'intérêt du sujet sur la base des premières valeurs, où la plage d'intérêt représente une partie de la région d'intérêt où au moins un motif prédéfini ou une caractéristique prédéfinie doit être attendu;
d) Réalisation d'un deuxième balayage de tomodensitométrie de la plage d'intérêt du sujet résultant en une deuxième image avec une deuxième résolution, où la deuxième résolution est supérieure à la première résolution; et
e) Application du même procédé de traitement d'imagerie médicale à la deuxième image résultant en des deuxièmes valeurs représentant une deuxième analyse de la plage d'intérêt du sujet, où le procédé de traitement d'imagerie médicale est configuré pour identifier une section de la deuxième image en détectant un motif prédéfini ou au moins une caractéristique prédéfinie dans la deuxième image.

2. Procédé selon l'une quelconque des revendications précédentes, dans lequel le premier balayage de densitométrie est réalisé avec une première dose de rayons X et le deuxième balayage de densitométrie est réalisé avec une deuxième dose de rayons X, où la deuxième dose de rayons X est supérieure à la première dose de rayons X.

3. Procédé selon la revendication précédente, dans lequel le procédé de traitement d'imagerie médicale comprend au moins l'une des sous-étapes suivantes:
- Extraction des caractéristiques de la première image;
- Classification des caractéristiques extraites;
- Détermination d'une entropie de la région d'intérêt du sujet;
- Détermination d'une entropie de la plage d'intérêt du sujet;
- Détermination d'une non-uniformité de la région d'intérêt du sujet; et
- Détermination d'une non-uniformité de la plage d'intérêt du sujet.

4. Procédé selon la revendication précédente, dans lequel les premières valeurs représentant la première analyse comprennent au moins une valeur représentant une caractéristique extraite, au moins une valeur représentant une caractéristique classifiée, au moins une valeur représentant une entropie déterminée et/ou au moins une valeur représentant une non-uniformité déterminée.

5. Procédé selon l'une quelconque des revendications 3 ou 4 précédentes, dans lequel les premières valeurs représentant la première analyse comprennent au moins une valeur représentant une sous-région de la région d'intérêt,
si une caractéristique de la sous-région a été déterminée,
et/ou si la caractéristique de la sous-région a été classée, notamment par rapport à une classe ou un type prédéfini,
et/ou si une entropie d'au moins une valeur d'entropie prédéfinie a été déterminée pour la sous-région,
et/ou si une non-uniformité d'au moins un degré prédéfini de non-uniformité a été déterminée pour ladite sous-région.

6. Procédé selon l'une quelconque des revendications 3 à 5 précédentes, dans lequel les deuxièmes valeurs représentant la deuxième analyse comprennent au moins une valeur représentant une caractéristique extraite, au moins une valeur représentant une caractéristique classifiée, au moins une valeur représentant une entropie déterminée et/ou au moins une valeur représentant une non-uniformité déterminée.

7. Procédé selon l'une quelconque des revendications précédentes, dans lequel la première résolution se réfère à 8 à 10 paires de lignes par centimètre (lp/cm) et/ou dans lequel la deuxième résolution se réfère à 11 à 24 paires de lignes par centimètre (lp/cm).

8. Procédé selon l'une quelconque des revendications précédentes, dans lequel le procédé de traitement de l'image médicale appliqué à l'étape b) à la première image comprend les sous-étapes:
- Identification d'au moins une première lésion au niveau de la première image, chaque première lésion représentant une tumeur;
- Détermination d'une première périphérie de chaque première lésion, où chaque première périphérie représente une région active de la tumeur respective; et
- Détermination des premières valeurs sur la base de l'au moins une première périphérie.

9. Procédé selon l'une quelconque des revendications précédentes, dans lequel le procédé de traitement d'image médicale appliqué à l'étape e) à la deuxième image comprend les sous-étapes:
- Identification d'au moins une deuxième lésion au niveau de la deuxième image, où chaque deuxième lésion représente une tumeur;
- Détermination d'une deuxième périphérie de chaque deuxième lésion, où chaque deuxième périphérie représente une région active de la tumeur respective; et
- Détermination des deuxièmes valeurs sur la base de l'au moins une deuxième périphérie.

10. Procédé selon la revendication précédente, dans lequel l'étape c) comprend les sous-étapes:
- Détermination pour chaque première lésion d'une sous-plage à l'intérieur de la région d'intérêt du sujet, de telle sorte que chaque sous-plage représente la première périphérie, ou au moins une partie de celle-ci, de la première lésion respective; et
- Détermination de la plage d'intérêt du sujet sur la base de l'au moins une sous-plage.

11. Procédé selon la revendication précédente, dans lequel au moins une des sous-plages est une plage axiale, qui représente une zone de tissu métastatique, en particulier une zone maximale de tissu métastatique.

12. Procédé selon l'une quelconque des revendications précédentes, dans lequel le deuxième balayage de densitométrie est composé de plusieurs sous-balayages de densitométrie, en particulier d'au moins un sous-balayage de densitométrie pour chaque sous-plage.

13. Procédé selon l'une quelconque des revendications précédentes, dans lequel le deuxième balayage est un balayage de densitométrie à contraste dynamique amélioré.

14. Procédé selon l'une quelconque des revendications précédentes, comprenant en outre une étape de segmentation entre les étapes a) et b), où ladite étape de segmentation consiste à segmenter manuellement ou automatiquement un volume à analyser dans la région d'intérêt balayée, le procédé de traitement de l'image médicale de l'étape b) étant appliqué uniquement audit volume à analyser.

15. Procédé selon l'une quelconque des revendications précédentes,
dans lequel l'étape b) comprend les sous-étapes suivantes:
- Extraction d'une pluralité de plaques volumétriques de la première image, et
- Calcul d'une métrique de signification de chaque plaque, où les premières valeurs représentent les métriques de signification parmi la pluralité de plaques;
où l'étape c) comprend les sous-étapes suivantes:
- Détermination de la plaque parmi la pluralité de plaques pour laquelle la métrique de signification la plus élevée a été calculée; et
- Détermination de la plage d'intérêt du sujet sur la base des premières valeurs, de sorte que la plage d'intérêt corresponde à au moins une partie de la plaque pour laquelle la métrique de signification la plus élevée a été calculée.

16. Système d'analyse d'image médicale, comprenant:
- un scanner CT;
- une unité de commande; et
- une unité de traitement;
où l'unité de commande est configurée pour commander le scanner CT, de sorte que le scanner CT effectue un premier balayage de tomodensitométrie d'une région d'intérêt d'un sujet résultant en une première image avec une première résolution;
où l'unité de traitement est configurée pour appliquer un procédé de traitement d'image médicale à la première image résultant en des premières valeurs représentant une première analyse de la région d'intérêt du sujet, où le procédé de traitement d'image médicale est configuré pour identifier une section de la première image en détectant un motif prédéfini ou au moins une caractéristique prédéfinie dans la première image;
où l'unité de traitement est configurée pour déterminer une plage d'intérêt du sujet sur la base des premières valeurs où la plage d'intérêt représente une partie de la région d'intérêt où au moins un motif prédéfini ou une caractéristique prédéfinie doit être attendu;
où l'unité de commande est configurée pour commander le scanner CT, de telle sorte que le scanner CT effectue un deuxième balayage de tomodensitométrie de la plage d'intérêt du sujet résultant en une deuxième image avec une deuxième résolution, où la deuxième résolution est supérieure à la première résolution ; et
où l'unité de traitement est configurée pour appliquer le même procédé de traitement d'imagerie médicale à la deuxième image résultant en des deuxièmes valeurs représentant une deuxième analyse de la plage d'intérêt du sujet, où le procédé de traitement d'imagerie médicale est configuré pour identifier une section de la deuxième image en détectant un motif prédéfini ou au moins une caractéristique prédéfinie dans la deuxième image.

17. Elément de programme informatique pour commander le système selon les revendications 16, qui, lorsqu'il est exécuté par une unité de traitement, est adapté pour exécuter les étapes de procédé selon l'une quelconque des revendications 1 à 15.

18. Support lisible par ordinateur ayant stocké l'élément de programme selon la revendication 17.
